Europäisches Patentamt

European Patent Office

Office européen des brevets

(19)

(11) Publication number:

**0 092 613
B1**

(12) **EUROPEAN PATENT SPECIFICATION**

(45) Date of publication of patent specification: **19.02.86**

(51) Int. Cl.⁴: **C 07 C 69/753,** C 07 C 67/56

(21) Application number: **82302057.3**

(22) Date of filing: **22.04.82**

(54) **Process for separating esters of fatty and rosin acids.**

(43) Date of publication of application:
**02.11.83 Bulletin 83/44**

(45) Publication of the grant of the patent:
**19.02.86 Bulletin 86/08**

(84) Designated Contracting States:
**AT BE CH DE FR GB IT LI NL SE**

(56) References cited:
**None**

(73) Proprietor: **UOP Inc.
10 UOP Plaza Algonquin & Mt. Prospect Roads
Des Plaines Illinois 60016 (US)**

(72) Inventor: **Cleary, Michael Terence
281 East Wilson
Elmhurst Illinois 60126 (US)**
Inventor: **Kulprathipanja, Santi
3920 Winston Drive
Hoffman Estates Illinois 60172 (US)**
Inventor: **Neuzil, Richard William
527 Eldon Place
Downers Grove Illinois 60515 (US)**

(74) Representative: **Hale, Stephen Geoffrey
J.Y. & G.W. Johnson Furnival House 14/18 High
Holborn
London WC1V 6DE (GB)**

Courier Press, Leamington Spa, England.

EP 0 092 613 B1

## Description

The present invention relates to a solid-bed adsorptive separation process for separating esters of fatty acids from esters of rosin acids.

It is well known in the separation art that certain crystalline aluminosilicates can be used to separate hydrocarbon types from mixtures thereof. For example, a separation process disclosed in U.S. 2,985,589 and 3,201,491 uses a type A zeolite to separate normal paraffins from branched chain paraffins, and processes described in U.S. 3,265,750 and 3,510,423 use type X or type Y zeolites to separate olefinic hydrocarbons from paraffinic hydrocarbons. In addition to their use in processes for separating hydrocarbon types, X and Y zeolites have been employed in processes to separate individual hydrocarbon isomers. For example, adsorbents comprising X and Y zeolites are used in the process described in U.S. 3,114,782 to separate alkyl-trisubstituted benzene isomers; in the process described in U.S. 3,864,416 to separate alkyl - tetrasubstituted monocyclic aromatic isomers; and in the process described in U.S. 3,668,267 to separate specific alkyl-substituted naphthalenes. Because of the commercial importance of para-xylene, perhaps more well-known and extensively used hydrocarbon isomer separation processes are those for separating para-xylene from a mixture of $C_8$-aromatics. In processes described in U.S. 3,558,730; 3,558,732; 3,626,020; 3,663,638 and 3,734,974, for example, adsorbents comprising particular zeolites are used to separate para-xylene from feed mixtures comprising para-xylene and at least one other xylene isomer by selectively adsorbing para-xylene compared to the other xylene isomers.

In contrast, this invention relates to the separation of non-hydrocarbons and more specifically to the separation of fatty acid esters from rosin acid esters.

Production of fatty esters is the most important phase in the industrial chemistry of fatty acids. The esters produced are of several types and include those resulting from the reaction of fatty acids with monohydric alcohols, polyhydric alcohols, ethylene or propylene oxide, and acetylene or vinyl acetate. The principal monohydric alcohols are methanol, 1-propanol, 2-propanol and 1-butanol. The greatest uses of esters are in the solvent and plasticizer fields. Esters of monohydric alcohols are used for plasticizers and in cosmetics. Esters of saturated fatty acids are of value in compound lubricating oil, as a lubricant for the textile and molding trade, in special lacquers, as a waterproofing agent, and in the cosmetic and pharmaceutical fields. Esters of unsaturated fatty acids find use as drying agents.

It is known from U.S. 4,048,205; 4,049,688 and 4,213,913 to use type X and type Y zeolites for the separation of unsaturated from saturated esters of fatty acids. We have found that the type X and type Y zeolites, however, will not separate the esters of fatty acids from a mixture thereof with esters of rosin acids such as is found in tall oil after esterification, apparently because the pore sizes of these zeolites (over 7 angstroms) are large enough to accommodate and retain the relatively large diameter molecules of esters of rosin acids as well as the smaller diameter molecules of esters of fatty acids. Type A zeolite, on the other hand, has a pore size (about 5 angstroms) which is unsable to accommodate either of the above type esters and is, therefore, unable to separate them.

We have discovered that silicalite, a non-zeolitic hydrophobic crystalline silica molecular sieve, is uniquely suitably for the adsorptive separation of an ester of a fatty acid from a mixture thereof with an ester of a rosin acid.

According to the present invention, there is provided a process for separating an ester of a fatty acid from a mixture comprising an ester of a fatty acid and an ester of a rosin acid, characterised in that the mixture is contacted at adsorption conditions with an adsorbent comprising silicalite, thereby selectively adsorbing the fatty acid ester thereon. The adsorbed fatty acid ester may be recovered by contacting the adsorbant containing it with a desorbent material in the absence of the feed mixture.

In one embodiment, the process is characterised by a combination of the steps of

(a) maintaining net fluid flow in a single direction through a column of the adsorbent comprising silicalite, said column containing at least three zones having separate operational functions occurring therein and being serially interconnected to provide a cyclic fluid flow path through the column;

(b) maintaining in the column an adsorption zone comprising the adsorbent located between a feed input port at its upstream boundary and a raffinate output port at its downstream boundary;

(c) maintaining a purification zone in the column immediately upstream from the adsorption zone and comprising the adsorbent located between an extract output port at its upstream boundary and the feed input port at its downstream boundary;

(d) maintaining a desorption zone in the column immediately upstream from the purification zone and comprising the adsorbent located between a desorbent input port at its upstream boundary and the extract output port at its downstream boundary;

(e) passing the feed mixture through the feed input port into the adsorption zone at adsorption conditions to effect the selective adsorption of the fatty acid ester by the adsorbent in the adsorption zone and withdrawing a raffinate output stream comprising the rosin acid ester from the raffinate output port of said desorption zone;

(f) passing the desorbent material through the desorbent input port into the desorption zone at desorption conditions to effect the displacement of the fatty acid ester from the adsorbent in the desorption zone, and withdrawing from the extract output port an extract output stream comprising the fatty acid ester and desorbent material; and

(g) periodically advancing along the cyclic flow path through the column in a downstream direction with respect to fluid flow the feed input port, raffinate output port, desorbent input port, and extract output port to effect the shifting of zones through the adsorbent and the continuous production of extract and raffinate output streams.

Optionally in such an embodiment a buffer zone is maintained in the column immediately upstream from the desorption zone, said buffer zone comprising the adsorbent located between the desorbent input port at its downstream boundary, and the raffinate output port at its upstream boundary.

In the context of the present invention, a "port" is a fluid access point or line where fluid can enter or leave a zone containing adsorbent; for convenience herein, locations of particular ports are sometimes defined by reference to the stream passing therethrough. A "feed mixture" is a mixture containing one or more extract components and one or more raffinate components to be separated by our process. An "extract component" is a compound of compound that is more selectively adsorbed by the adsorbent while a "raffinate component" is a compound or type of compound that is not absorbed or less selectively adsorbed. In this invention the fatty acid ester is an extract component and the rosin acid ester is a raffinate component. The term "desorbent material" means a material capable of desorbing the extract component from the adsorbent. The terms "raffinate output stream" and "extract output stream" mean streams through which a raffinate component and an extract component are removed from the system. The composition of the raffinate output stream and extract output stream can vary from essentially 100% desorbent material to essentially 100% raffinate component or extract component, as the case may be. At least a portion of the extract output stream and preferably also at least a portion of the raffinate output stream are usually passed to separation means, typically a fractionator, where at least a portion of the desorbent material is separated to produce an extract product or a raffinate product as the case may be. The terms "extract product" and "raffinate product" mean products produced by the process and containing, respectively, an extract component and a raffinate component in higher concentrations than those found in the extract output stream and the raffinate output stream. Although it is possible by the process of this invention to produce a high purity, fatty acid ester product or rosin acid ester product (or both) at high recoveries, it will be appreciated that an extract component is never completely adsorbed by the adsorbent, nor is a raffinate component completely non-adsorbed by the adsorbent.

Therefore, varying amounts of raffinate component can appear in the extract output stream and, likewise, varying amounts of an extract component can appear in the raffinate output stream. The extract and raffinate streams then are further distinguished from each other and from the feed mixture by the ratio of the concentration of extract component and raffinate component appearing in the particular stream. More specifically, the ratio of the concentration of fatty acid ester to that of less selectively adsorbed rosin acid ester will be lowest in the raffinate output stream, next highest in the feed mixture, and the highest in the extract output stream.

Before considering feed mixtures which can be used to the process of this invention brief reference is first made to the terminology and to the general production of fatty acids used to make the fatty acid esters. The fatty acids are a large group of aliphatic monocarboxylic acids, many of which occur as glycerides (esters or glycerol) in natural fats and oils. Although the term "fatty acids" has been restricted by some to the saturated acids of the acetic acid series, both normal and branched chain, it is now generally used, and is so used herein, to include also related unsaturated acids, certain substituted acids, and even aliphatic acids containing alicyclic substituents. The naturally occuring fatty acids with a few exceptions are higher straight chain unsubstituted acids containing an even number of carbon atoms. The unsaturated fatty acids can be divided, on the basis of the number of double bonds in the hydrocarbon chain, into mono-ethanoid, diethanoid, triethanoid, etc. (or mono-ethylenic, etc.). Thus the term "unsaturated fatty acid" is a generic term for a fatty acid having at least one double bond, and the term "poly-ethanoid fatty acid" means a fatty acid having more than one double bond per molecule. Fatty acids are typically prepared from glyceride fats or oils by one of several "splitting" or hydrolytic processes. In all cases the hydrolysis reaction may be summarized as the reaction of a fat or oil with water to yield fatty acids plus glycerol. In modern fatty acid plants this process is carried out by continuous high pressure, high temperature hydrolysis of the fat. Starting materials commonly used for the production of fatty acids include coconut oil, palm oil, inedible animal fats, and the commonly used vegetable oils, soybean oil, cottonseed oil and corn oil.

The source of fatty acids with which the present invention is primarily concerned is tall oil, a by-product of the wood pulp industry, usually recovered from pine wood "black liquor" of the sulfate or kraft paper process. Tall oil contains about 50—90% by weight fatty acids and about 20—40% by weight rosin acids. The fatty acids include oleic, linoleic, palmitic and stearic acids. Rosin acids are monocarboxylic acids having the molecular formula $C_{20}H_{30}O_2$ and a molecular structure comprising fused rings, which accounts for the much larger molecular diameter of rosin acids as compared to fatty acids. The acids contained in the feed mixtures to the present process are typically esterified according to any one of the known procedures. Esterification with a monohydric alcohol to form the corresponding methyl, ethyl, propyl, n-butyl, etc., ester is particularly preferred, especially the methyl and ethyl esters of these acids. Feed mixtures which can be charged to the process may contain, in addition to esters of the components of tall oil, a diluent material that is not adsorbed by the adsorbent and which is preferably separable from the extract and raffinate output streams by fractional distillation. Paraffinc hydrocarbons are examples of suitable diluents. Cyclohexane is one specific example of a paraffinic hydrocarbon that can be used as a diluent. When a diluent is employed the concentration of the diluent in the mixture of diluent and esters will preferably be from a few vol. % up to 75 vol. % with the remainder being fatty acid esters and rosin acid esters.

Desorbent materials used in adsorptive separation processes vary depending upon such factors as the type of operation employed. In the swing-bed system in which a selectively adsorbed feed component is removed from the adsorbent by a purge stream desorbent selection is not as critical and desorbent materials comprising gaseous hydrocarbons such as methane or ethane or other types of gases such as nitrogen or hydrogen may be used at elevated temperatures or reduced pressures or both to effectively purge the adsorbed feed component from the adsorbent. However, in adsorptive separation processes which are generally operated continuously at substantially constant pressures and temperatures to ensure liquid phase, a desorbent material must be judiciously selected to satisfy many criteria. First, a desorbent material should displace an extract component from an adsorbent with reasonable mass flow rates without itself being so strongly adsorbed as to unduly prevent an extract component from displacing the desorbent material in a following adsorption cycle. Expressed in terms of the selectively, it is preferred that an adsorbent be more selective for all extract components with respect to a raffinate component than it is for a desorbent material with respect to a raffinate component. Secondly, desorbent materials must be compatible with a particular adsorbent and a particular feed mixture. More specifically, a desorbent material must not reduce or destroy the critical selectivity of an adsorbent for an extract component with respect to a raffinate component. A desorbent material should additionally be a substance which is easily separable from the feed mixture that is passed into the process. A raffinate stream and an extract stream removed from an adsorbent both typically contain desorbent material and without a method of separating at least a portion of the desorbent material the purity of an extract product and a raffinate product would not be very high, nor would a desorbent material be available for reuse in the process. It is therefore contemplated that any desorbent material used in this process will preferably have an average boiling point substantially different from that of a feed mixture to allow separation of at least a portion of desorbent material from feed components in an extract and a raffinate stream by simple fractional distillation, thereby permitting reuse of a desorbent material in the process. The term "substantially different" as used herein means that the difference in the average boiling points between a desorbent material and a feed mixture is at least 5°C. The boiling range of a desorbent material may be higher or lower than that of a feed mixture. Finally, a desorbent material should also be a material which is readily available and therefore reasonable in cost. In the process of the present invention, we have found that desorbent materials comprising paraffins, especially those having average boiling points substantially different from (i.e. 5°C or more above or below) that of the feed mixture meet these requirements and are particularly effective. Cyclohexane is a particularly preferred desorbent for a feedstock comprising esters of tall oil ingredients.

The prior art has also recognised that certain characteristics of adsorbents are highly desirable, if not absolutely necessary, to the successful operation of a selective adsorption process. In this connection attention is directed to the passage in GB—A—2049667 from page 5 line 26 to page 6 line 61.

The adsorbent to be used in the process of this invention comprises silicalite. As previously mentioned, silicalite is a hydrophobic crystalline silica molecular sieve. Due to its aluminum-free structure, silicalite does not show ion-exchange behaviour, and is hydrophobic and organophilic. Silicalite thus comprises a molecular sieve but not a zeolite. Silicalite is uniquely suitable for the separation process of this invention for the presumed reason that its pores are of a size and shape that enable the silicalite to function as a molecular sieve, i.e. accept the molecules of esters of fatty acids into its channels or internal structure, while rejecting the molecules of esters of rosin acids. It is preferred that the alkali metal content of the silicalite material used in the present invention be held to a low value—less than 0.2 wt. being especially preferred. A detailed discussion of silicalite may be found in the article "Silicalite, a new hydrophobic crystalline silica molecular sieve", *Nature*, Vol. 271, 9 February 1978. The composition, method of preparation and characteristics of silicalite are also disclosed in U.S. 4,061,724. This material is available from the Linde Division of Union Carbide Corporation under the trademark "ELZ-115". The adsorbent may be used herein in either a bound or unbound form.

When used in a bound form, the adsorbent typically comprises silicalite composited with a matrix material or binder as support material. Silicalite will typically be present in this adsorbent composite in amounts of 5 to 98 wt. % thereof, with best results obtained in the range of 75 to 98 wt. % thereof, based on volatile free composition (i.e. after it is calcined at 600 to 900°C in order to drive off all volatile matter). The remainder of the adsorbent composite will generally be materials such as silica, alumina, or silica-alumina mixtures, such as clays, and the like well known matrix materials. A silica matrix material is ordinarily preferred. The matrix material or binder may also be a material such as graphite or cellulose acetate which during a subsequent calcination operation is essentially burned out of the composite in order to produce a bound silicalite material having the desired particle size.

The adsorbent may be employed in the form of a dense compact fixed bed which is alternatively contacted with the feed mixture and desorbent materials. In the simplest embodiment of the invention, the adsorbent is employed in the form of a single static bed in which case the process is only semi-continuous. In another embodiment a set of two or more static beds may be employed in fixed bed contacting with appropriate valving so that the feed mixture is passed through one or more adsorbent beds while the desorbent materials can be passed through one or more of the other beds in the set. The flow of feed mixture and desorbent materials may be either up or down through the desorbent. Any of the conventional apparatus employed in static bed fluid-solid contacting may be used. The particles of silicalite adsorbent can have any convenient form such as spheres, cylindrical extrudates and the like, and will preferably have a particle size range of 16—60 mesh (Standard U.S. Mesh) or about 250 to 1190 microns.

Countercurrent moving bed or simulated moving bed countercurrent flow systems, however, have a much greater separation efficiency than fixed adsorbent bed systems and are therefore preferred. In the moving bed or simulated moving bed processes the adsorption and desorption operations are continuously taking place which allows both continuous production of an extract and a raffinate stream and the continual use of feed and desorbent streams. One preferred embodiment of this process utilizes what is known in the art as the simulated moving bed countercurrent flow system. The operating principles and sequence of such a flow system are described in the passage at page 8 line 53—page 10 line 2 of GB—A—2049667 which passage is incorporated herein by reference.

It is contemplated that at least a portion of the extract output stream will pass into a separation means wherein at least a portion of the desorbent material can be separated to produce an extract product containing a reduced concentration of desorbent material. Preferably, but not necessary to the operation of the process, at least a portion of the raffinate output stream will also be passed to a separation means wherein at least a portion of the desorbent material can be separated to produce a desorbent stream which can be reused in the process and a raffinate product containing a reduced concentration of desorbent material. The separation means will typically be a fractionation column, the design and operation of which is well known to the separation art.

Reference can be made to U.S. 2,985,589, and to a paper entitled "Continous Adsorptive Processing—A New Separation Technique" by D. B. Broughton presented at the 34th Annual Meeting of the Society of Chemical Engineers at Tokyo, Japan on April 2, 1969, for further explanation of the simulated moving bed countercurrent process flow scheme.

Although both liquid and vapor phase operations can be used in many adsorptive separation processes, liquid-phase operation is preferred for this invention because of the lower temperature requirements and because of the higher yields of extract product that can be obtained with liquid-phase operation over those obtained with vapor-phase operation. Adsorption conditions typically include a temperature of from 20°C to 100°C, with 20°C to 175°C being preferred, and a pressure sufficient to maintain liquid-phase. Desorption conditions typically include the same range of temperatures and pressures as used for adsorption conditions.

The size of the units which can be utilized in the process of this invention can vary anywhere from those of pilot-plant scale (see for example U.S. 3,706,812) to those of commercial scale and can range in flow rates from as little as a few cc an hour up to many thousands of litres per hour.

The following example is presented to illustrate the selectivity relationship that makes the process of this invention possible.

Example

The pulse test apparatus described in GB—A—2049667 at page 6 lines 13—30 was used to obtain data for this example. The column was 70 cc, upflow, and helical. The liquid temperature was 160°C and the flow rate 1.2 ml/min. The column was packed with silicalite adsorbent. The adsorbent was prepared by calcining a sample of ELZ-115 silicalite material obtained from the Linde Co. at 600°C for about 2 hours until a faint pink color disappeared. The resulting adsorbent is believed to be essentially 100% silicalite containing less than 0.2 wt. % alkali metal and was used herein in an unbound form in a mesh size 20 to 50 U.S. Sieve Standard series (297 to 840 microns). The tall oil used in the example was obtained from Emery Industries Inc. under the registered trademark "EMTALL 731" and it is reported to have the typical characteristic shown in the following Table 1:

## TABLE 1
Typical specification for distilled tall oil

| | |
|---|---|
| Rosin Acid Content, vol. % | 30 |
| Fatty Acid Content, vol. % | 70 |
| Acid Value | 185 |
| Rosin Acid Numbers | 56 |
| Saporification Value | 186 |
| Iodine Value | 175 |
| Specific Gravity, 15°C | 0.951 |
| Specific Gravity, 32°C | 0.936 |
| Specific Gravity, 49°C | 0.927 |
| Ash, ppm | 1 |

The feed stream was made up of 20 vol. % distilled tall oil, 40 vol. % Methyl-8 reagent (mostly pyridine) and 40 vol. % cyclohexane. The Methyl-8 reagent is a solution of dimethylformamide dimethylacetols in pyridine available from Pierce Chemical Co. under the registered trademark "Methyl-8" and used for speedy and quantitative esterification of fatty acids and other carboxylic acids. The purpose of the Methyl-8 reagent was to form methyl esters of the fatty acids and rosin acids in the tall oil prior to contact of the feed with the adsorbent. The desorbent used was cyclohexane and it was added to the heated mixture from the esterification step. After mixing the Methyl-8 reagent with the tall oil, the resulting solution was maintained at 60°C for 10 to 15 minutes.

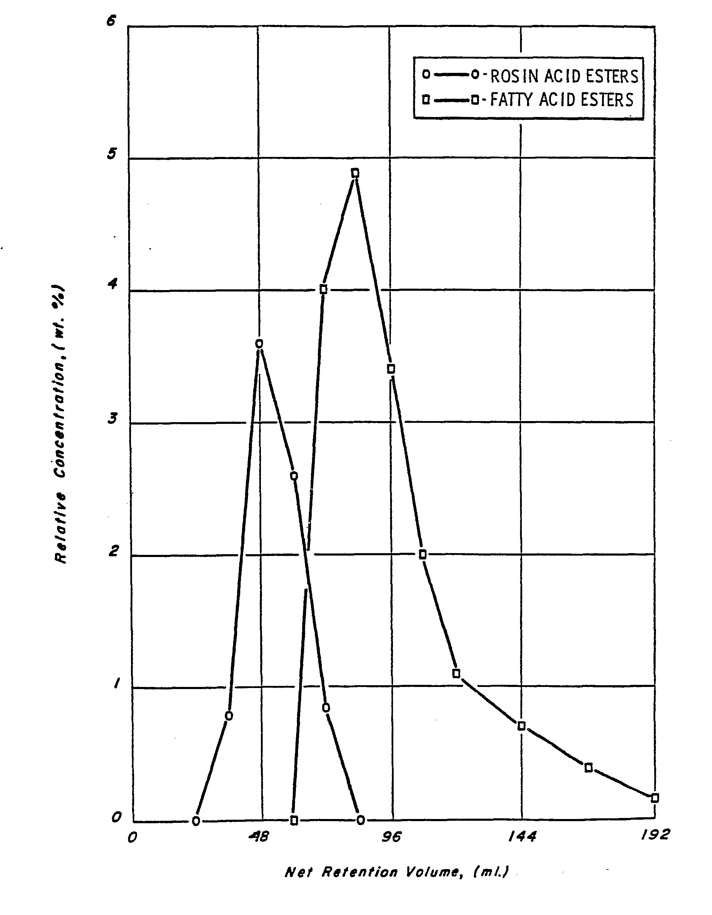

The results are as shown in the elution curves comprising the accompanying drawing. These curves clearly illustrate a remarkable separation of fatty acid esters from rosin acid esters from the standpoint of not only purity of the raffinate and extract products, but also the very high yield of pure products, i.e. the curves show a very small overlap or amount of volume eluted where the product is an undesirable mixture of the esters of both rosin and fatty acids.

## Claims

1. A process for separating an ester of a fatty acid form a feed mixture comprising an ester of a fatty acid and an ester of a rosin acid, characterised in that the feed mixture is contacted with an adsorbent comprising silicalite, thereby selectively adsorbing the fatty acid ester thereon.

2. A process as claimed in claim 1, characterised in that the fatty acid ester is recovered by contacting the adsorbent containing it with a desorbent material in the absence of the feed mixture.

3. A process as claimed in claim 1 or 2, characterised in that the feed mixture comprises esters of the fatty acid and rosin acid components of tall oil.

4. A process as claimed in claim 3, characterised in that the esters are methyl esters.

5. A process as claimed in claim 2 and claim 3 or claim 4, characterised in that the desorbent material is a paraffinic hydrocarbon.

6. A process as claimed in any of claims 1 to 5, characterised in that the feed mixture is contacted with the adsorbent at a temperature of from 20°C to 200°C and a pressure sufficient to maintain the feed mixture in liquid phase.

7. A process as claimed in claim 2 or in any of claims 3 to 6 as appendent to claim 2, characterised in that the adsorbent is contacted with the desorbent at from 20°C to 200°C and a pressure sufficient to maintain the desorbent in liquid phase.

8. A process as claimed in claim 2 or in claim 2 and any of claims 3 to 7, characterised by the combination of

(a) maintaining net fluid flow in a single direction through a column of the adsorbent comprising silicalite, said columns containing at least three zones having separate operational functions occuring therein and being serially interconnected to provide a cyclic fluid flow path through the column;

(b) maintaining in the column an adsorption zone comprising the adsorbent located between a feed input port at its upstream boundary and a raffinate output port at its downstream boundary;

(c) maintaining a purification zone in the column immediately upstream from the adsorption zone and comprising the adsorbent located between an extract output port at its upstream boundary and the feed input port at its downstream boundary;

(d) maintaining a desorption zone in the column immediately upstream from the purification zone and comprising the adsorbent located between a desorbent input port at its upstream boundary and the extract output port at its downstream boundary;

(e) passing the feed mixture through the feed input port into the adsorption zone at adsorption conditions to effect the selective adsorption of the fatty acid ester by the adsorbent in the adsorption zone and withdrawing a raffinate output stream comprising the rosin acid ester from the raffinate output port of said desorption zone;

(f) passing the desorbent material through the desorbent input port into the desorption zone at desorption conditions to effect the displacement of the fatty acid ester from the adsorbent in the desorption zone, and withdrawing from the extract output port an extract output stream comprising the fatty acid ester and desorbent material; and

(g) periodically advancing along the cyclic flow path through the column in a downstream direction with respect to fluid flow the feed input port, raffinate output port, desorbent input port, and extract output port to effect the shifting of zones through the adsorbent and the continous production of extract and raffinate output streams.

9. A process as claimed in claim 8, characterised in that a buffer zone is maintained in the column immediately upstream from the desorption zone, said buffer zone comprising the adsorbent located between the desorbent input port at its downstream boundary and the raffinate output port at its upstream boundary.

**Revendications**

1. Procédé pour séparer un ester d'un acide gras hors d'un mélange d'alimentation comprenant un ester d'un acid gras et un ester d'un acide résinique, caractérisé en ce que le mélange d'alimentation est mis en contact avec un adsorbant comprenant de la silicalite de manière à adsorber sélectivement l'ester d'acide gras sur celle-ci.

2. Procédé suivant la revendication 1, caractérisé en ce que l'ester d'acide gras est isolé par mise en contact de l'adsorbant le contenant avec une matière désorbante en l'absence du mélange d'alimentation.

3. Procédé suivant la revendication 1 ou 2, caractérisé en ce que le mélange d'alimentation comprend des esters des composants acides gras et acides résiniques du tallöl.

4. Procédé suivant la revendication 3, caractérisé en ce que les esters sont les esters méthyliques.

5. Procédé suivant la revendication 2 et la revendication 3 ou 4, caractérisé en ce que la matière désorbante est un hydrocarbure paraffinique.

6. Procédé suivant l'une quelconque des revendications 1 à 5, caractérisé en ce que le mélange d'alimentation est mis en contact avec l'adsorbant à une température de 20°C à 200°C et sous une pression suffisante pour maintenir le mélange d'alimentation en phase liquide.

7. Procédé suivant la revendication 2 ou l'une quelconque des revendications 3 à 6 en dépendance de la revendication 2, caractérisé en ce que l'adsorbant est mis en contact avec le désorbant de 20°C à 200°C sous une pression suffisante pour maintenir le désorbant en phase liquide.

8. Procédé suivant la revendication 2 ou la revendication 2 et l'une quelconque des revendications 3 à 7, caractérisé par la combinaison des stades

(a) d'entretenir un écoulement de fluide net dans une direction unique dans une colonne de l'adsorbant comprenant la silicalite, la colonne contenant au moins trois zones ayant des fonctions opérationnelles distinctes qui y ont lieu et étant interconnectées en série pour établir un trajet d'écoulement de fluide cyclique dans la colonne;

(b) d'entretenir dans la colonne une zone d'adsorption comprenant l'adsorbant situé entre un orifice d'admission d'alimentation à sa limite d'amont et un orifice de sortie de raffinat à sa limite d'aval;

(c) d'entretenir une zone de purification dans la colonne immédiatement à l'amont de la zone d'adsorption et comprenant l'adsorbant situé entre un orifice de sortie d'extrait à sa limite d'amont et l'orifice d'admission d'alimentation à sa limite d'aval;

(d) d'entretenir une zone de désorption dans la colonne immédiatement à l'amont de la zone de purification et comprenant l'adsorbant situé entre un orifice d'admission de désorbant à sa limite d'amont et l'orifice de sortie d'extrait à sa limite d'aval;

(e) de faire passer le mélange d'alimentation par l'orifice d'admission d'alimentation dans la zone d'adsorption dans des conditions d'adsorption pour opérer l'adsorption sélective de l'ester d'acide gras par l'adsorbant dans la zone d'adsorption et de prélever un courant de sortie de raffinat comprenant l'ester d'acide résinique par l'orifice de sortie de raffinat de la zone d'adsorption;

(f) de faire passula matière désorbante par l'orifice d'admission de désorbant dans la zone de désorption dans des conditions de désorption pour opérer le déplacement de l'ester d'acide gras hors de l'adsorbant dans la zone de désorption et de prélever par l'orifice de sortie d'extrait un courant de sortie d'extrait comprenant l'ester d'acide gras et la matière désorbante, et

(g) de faire avancer périodiquement au long du trajet d'écoulement cyclique dans la colonne en direction d'aval par rapport à l'écoulement de fluide l'orifice d'admission d'alimentation, l'orifice de sortie de raffinat, l'orifice d'admission de désorbant et l'orifice de sortie d'extrait pour opérer le mouvement des zones dans l'adsorbant et la production continue de courants de sortie d'extrait et de raffinat.

9. Procédé suivant la revendication 8, caractérisé en ce qu'une zone tampon est entretenue dans la colonne immédiatement à l'amont de la zone de désorption, la zone tampon comprenant l'adsorbant situé entre l'orifice d'admission de désorbant à sa limite d'aval et l'orifice de sortie de raffinat à sa limite d'amont.

**Patentansprüche**

1. Verfahren zur Abtrennung eines Fettsäureesters aus einem einen Fettsäureester und einen Harzsäureester enthaltenden Einsatzgemisch, dadurch gekennzeichnet, dass man das Einsatzgemisch mit einem aus Silicalit bestehenden Adsorptionsmittel in Berührung bringt und dadurch den Fettsäureester selektiv darauf adsorbiert.

2. Verfahren nach Anspruch 1, dadurch gekennzeichnet, dass man den Fettsäureester durch Inberührungbringen des ihn enthaltenden Adsorptionsmittels mit einem Desorptionsmaterial in Abwesenheit des Einsatzgemischs zurückgewinnt.

3. Verfahren nach Anspruch 1 oder 2, dadurch

13 **0 092 613** 14

gekennzeichnet, dass das Einsatzgemisch aus den Fettsäure- und Harzsäureesterkomponenten aus Tallöl besteht.

4. Verfahren nach Anspruch 3, dadurch gekennzeichnet, dass die Ester Methylester sind.

5. Verfahren nach Anspruch 2 und 3 oder Anspruch 4, dadurch gekennzeichnet, dass das Desorptionsmaterial ein Paraffinkohlenwasserstoff ist.

6. Verfahren nach einem der Ansprüche 1 bis 5, dadurch gekennzeichnet, dass das Einsatzgemisch bei einer Temperatur von 20°C bis 200°C und einem ausreichenden Druck, um das Einsatzgemisch in flüssiger Phase zu halten, mit dem Adsorptionsmittel in Berührung gebracht wird.

7. Verfahren nach Anspruch 2 oder einem der Ansprüche 3 bis 6, soweit sich diese auf Anspruch 2 beziehen, dadurch gekennzeichnet, dass das Adsorptionsmittel mit dem Desorptionsmittel bei 20°C bis 200°C und einem ausreichenden Druck, um das Desorptionsmittel in flüssiger Phase zu halten, in Berührung gebracht wird.

8. Verfahren nach Anspruch 2 oder Anspruch 2 und einem der Ansprüche 3 bis 7, dadurch gekennzeichnet, dass man in Kombination

a) eine Nettoflüssigkeitsströmung in einer einzigen Richtung durch eine Säule des aus Silicalit bestehenden Adsorptionsmittels aufrechterhält, wobei diese Säule mindestens drei Zonen aufweist, in denen getrennte Betriebsfunktionen ablaufen, und die so in Reihe geschaltet sind, dass ein zyklischer Flüssigkeitsströmungsweg durch die Säule entsteht,

b) in der Säule eine Adsorptionszone aufrechterhält, die das Adsorptionsmittel zwischen einer Zufuhröffnung an ihrer stromaufwärtigen Begrenzung und einer Raffinatauslassöffnung an ihrer stromabwärtigen Begrenzung angeordnet enthält,

c) unmittelbar stromaufwärts der Adsorptionszone in der Säule eine Reinigungszone aufrechterhält, die das Adsorptionsmittel zwischen einer Extraktauslassöffnung an ihrer stromaufwärtigen Begrenzung und der Zufuhröffnung an ihrer stromabwärtigen Begrenzung angeordnet enthält,

d) unmittelbar stromaufwärts der Reinigungszone in der Säule eine Desorptionszone aufrechterhält, die das Adsorptionsmittel zwischen einer Desorptionsmitteleinlassöffnung an ihrer stromauswärtigen Begrenzung und der Extraktauslassöffnung an ihrer stromabwärtigen Begrenzung angeordnet enthält,

e) das Einsatzgemisch durch die Zufuhröffnung in die Adsorptionszone unter Adsorptionsbedingungen leitet, um eine selektive Adsorption des Fettsäureesters durch das Adsorptionsmittel in der Adsorptionszone zu bewirken, und einen den Harzsäureester enthaltenden Raffinatausgangsstrom von der Raffinatauslassöffnung jener Adsorptionszone abzieht,

f) das Desorptionsmaterial durch die Desorptionsmitteleinlassöffnung in die Desorptionszone unter Desorptionsbedingungen einleitet, um eine Verdrängung des Fettsäureesters aus dem Adsorptionsmittel in der Desorptionszone zu bewirken, und einen den Fettsäureester und Desorptionsmaterial enthaltenden Extraktauslassstrom von der Extraktauslassöffnung abzieht und

g) von Zeit zu Zeit die Zufuhröffnung, die Raffinatauslassöffnung, die Desorptionsmitteleinlassöffnung und die Extraktauslassöffnung stromabwärts entlang dem zyklischen Strömungsweg durch die Säule vorschiebt, um eine Verschiebung der Zonen durch das Adsorptionsmittel und kontinuierliche Produktion von Extrakt- und Raffinatausgangsströmen zu bewirken.

9. Verfahren nach Anspruch 8, dadurch gekennzeichnet, dass man in der Säule unmittelbar stromaufwärts der Desorptionszone eine Pufferzone aufrechterhält, welche das Adsorptionsmittel zwischen der Desorptionsmitteleinlassöffnung an ihrer stromabwärtigen Begrenzung und der Raffinatauslassöffnung an ihrer stromaufwärtigen Begrenzung angeordnet enthält.

# 0 092 613

Net Retention Volume, (ml.)